# EUROPEAN PATENT APPLICATION

(11) **EP 4 799 677 A2**
(43) Date of publication of application: **02.09.2026**
(21) Application number: 26193468.1
(22) Date of filing: 15.12.2021
(51) Int. Cl.: A61N 1/36

(54) **IMPLANT DELIVERY DEVICE**

(30) Priority: 15.12.2020 EP 20214028
(62) Document number(s) of the earlier application(s) in accordance with Art. 76 EPC: 21830690.0 / 4 262 589
(71) Applicant: Capri Medical Limited, Dublin D04 W2K0 (IE)
(72) Inventor: MULLINS, John, Dublin (IE); WARD, Fergal, Dublin (IE); HEGARTY, Dominic, Dublin (IE); JONES, Aiden, Dublin (IE)
(74) Representative: HGF

(57) **Abstract**

The present application provides an implant delivery device (10) for implanting a medical implant (1) in a patient. The medical implant (1) comprises an implant housing (2) and an elongate electrode lead (3) extending from the implant housing. The implant delivery device (10) comprises a handle (11), a first needle (12) and a second needle (13). The first needle (12) is fixed to the handle (11) and comprises a lumen adapted to receive the implant housing (2) of the medical implant (1). The second needle (13) has a higher gauge than the first needle (12) and is adapted to carry the elongate electrode lead (3). The second needle (13) is retractably mounted to the handle (11) such that the second needle (13) is movable between: an extended position in which the second needle (13) extends beyond the first needle (12), and a retracted position in which the second needle (13) is retracted towards the handle (11) to release the electrode lead (3) from the second needle (13). The implant delivery device further comprises a locking mechanism (17) adapted to lock the second needle in the extended position.

## Description

This invention relates to an implant delivery device for implanting a medical implant, such as a neuromodulation implant, in a patient.

### BACKGROUND

It is known to provide an implantable neurostimulator comprising a housing and an electrode. A power antenna, microcontroller, and communication antenna are disposed in the housing for receiving power from an external source and receiving/transmitting sensor information relating to the electrode. A delivery system can be used to position the neurostimulator in a patient, in particular proximate to a nerve, by cutting an opening in the patient and passing the delivery system into the opening to position the implantable neurostimulator.

### BRIEF SUMMARY OF THE DISCLOSURE

In accordance with the present disclosure there is provided an implant delivery device for implanting a medical implant in a patient, the medical implant comprising an implant housing and an elongate electrode lead extending from the implant housing, and wherein the implant delivery device comprises:
a handle,
a first needle fixed to the handle and extending in a longitudinal direction, the first needle comprising a lumen adapted to receive the implant housing of the medical implant, and
a second needle having a higher gauge than the first needle, the second needle being adapted to carry the elongate electrode lead of the medical implant, and wherein the second needle is retractably mounted to the handle such that the second needle is movable between:
   an extended position in which the second needle extends beyond the first needle in the longitudinal direction such that during use the first needle and the second needle can simultaneously position the implant housing at a first depth and the electrode lead at a second depth greater than the first depth, and
   a retracted position in which the second needle is retracted towards the handle to release the electrode lead from the second needle.

In examples, the first needle and the second needle each comprise a sharp tip, for example a bevel tip, adapted to pierce the skin and penetrate the tissue of the patient during use to position the first needle and the second needle for releasing the electrode. In examples, the first needle is configured to only penetrate the skin and underlying fatty tissue during use. Accordingly, the first needle, which is larger, only penetrates the patient to the minimum required depth and reduces tissue damage.

In examples, the second needle comprises a lumen adapted to receive the elongate electrode lead of the medical implant. In examples, the second needle comprises a slot extending along one side and extending into the lumen. The slot may extend to a tip of the second needle. The slot may extend from the tip of the second needle into the lumen of the first needle when the second needle is in the extended position. In examples, the first needle and the second needle are non-concentrically arranged such that a portion of the electrode lead extends from the lumen of the second needle through the slot to the implant housing during use.

In examples, the second needle comprises an attachment point at or near a distal end of the second needle, and wherein the attachment point is adapted to attach to the elongate electrode lead such that the elongate electrode lead is carried along a side of the second needle. For example, the attachment point may comprise a hole adapted to receive an end of the elongate electrode lead. The hole may be formed in the distal end of the second needle, or in a side of the second needle proximate to the distal end.

In examples, the second needle is at least partly disposed in the lumen of the first needle. For example, the second needle extends alongside the implant housing within the lumen of the first needle. In examples, the lumen of the first needle may comprise a primary portion adapted to receive the implant housing and a secondary portion adapted to receive the second needle, the primary portion and the secondary portion may each comprise a partially circular cross-section. The first needle may have a tear drop cross-sectional profile. In examples, the cross-sectional area of the primary and secondary portions are approximately circular and arranged to partially overlap.

In examples, the implant housing is arranged in the lumen of the first needle such that the implant housing can be pulled out of the lumen of the first needle. Accordingly, a holding force provided between the electrode lead and the tissue of the patient after the electrode lead has been released will pull the implant housing out of the lumen of the first needle as the implant delivery device is removed from the patient.

In examples, the implant delivery device further comprises a retaining member adapted to releasably secure the implant housing in the lumen of the first needle. The retaining member may be operable by an operator to release the implant housing and allow the implant housing to move out of the first needle and into a deployed position in the patient.

In examples, the retaining member may comprise a retaining tab formed in a wall of the first needle and adapted to engage the implant housing in the lumen of the first needle. The retaining tab may be formed by a U-shaped cut in the first needle that is partially deformed into the lumen of the first needle. The implant housing may include a recess arranged to correspond with the retaining tab. The first needle may include multiple retaining tabs distributed longitudinally and/or circumferentially about the first needle and adapted to engage the implant housing.

In examples, the implant housing may comprise an attachment point, for example a hole. The retaining member may extend from a part of the handle to the attachment point of the implant housing, and the retaining member may be detachable to release the implant housing.

In examples, the retaining member may comprise a thread that is looped through the attachment point of the implant housing, and the thread may be split by the operator, for example cut or untied, to release the implant housing. In examples, the thread may comprise a suture, for example comprising Nylon, Monocryl, Vicryl, silk, or Prolene, or a metallic wire such as steel wire, or other material such as Kevlar.

In examples, the retaining member may comprise a shape memory member having a hooked end adapted to engage the attachment point of the implant housing. An opposite end of the shape memory housing may be arranged to be moved to deform the hooked end to release the implant housing. The opposite end of the shape memory member may be attached to a sliding tab arranged to be slid by an operator to deform the hooked end.

In examples, the retaining member comprises a clamping cap adapted to be push-fit onto an end of the implant housing. The implant delivery device may further comprise a spring-loaded release mechanism operable to separate the clamping cap from the implant housing.

In examples, the implant delivery device may further comprise an actuation tab arranged adjacent to the handle. The actuation tab may be attached to, or part of, the second needle. Alternatively, the actuation tab may be operably coupled to the second needle. The actuation tab may be configured for actuation by a user to move the second needle into the retracted position. In some examples, the actuation tab may be movable in a direction away from the handle and away from the patient to move the second needle into the retracted position. In other examples, the actuation tab may be moveable in a direction towards the handle and patient to move the second needle into the retracted position.

In examples, the implant delivery device may further comprise a locking mechanism adapted to lock the second needle in the extended position. The locking mechanism may directly lock the second needle in the extended position, or the locking mechanism may lock the actuation tab such that the second needle is also locked in the extended position.

In examples, the implant delivery device comprises a locking slot in the handle. The second needle may be adapted to engage the locking slot such that in one rotational position of the second needle relative to the handle the second needle is locked in the axial direction relative to the handle, and in another rotational position of the second needle relative to the housing the second needle is movable relative to the handle in an axial direction. In examples, the locking slot may comprise an L-shaped or J-shaped slot. In examples, the handle may comprise a locking ring and the locking slot may be formed in the locking ring.

In examples, the actuation tab is moveable in a first direction to move the second needle in a second direction opposite to the first direction. The implant delivery device may additionally comprise a rack and pinion mechanism operably coupled between the actuation tab and the second needle.

In examples, the handle may comprise a guide member and the actuation tab may be movable along the guide member. A locking mechanism may be configured to lock the actuation tab at a position along the guide member. The locking mechanism may comprise a button arranged to release the locking mechanism for movement of the actuation tab along the guide member.

In examples, the implant delivery device may additionally comprise a retaining member adapted to releasably secure the implant housing in the lumen of the first needle, and the retaining member may be actuated by the actuation tab to release the implant housing. In some examples, movement of the actuation tab through a first distance in a first direction moves the second needle to the retracted position, and further movement of the actuation tab through a second distance in the first direction actuates the retaining member to release the implant housing. In examples, the retaining member comprises a clamping cap adapted to be push-fit onto the implant housing, and the implant delivery device may further comprise a spring-loaded release mechanism operable to separate the clamping cap from the implant housing when actuated by the actuation tab. In particular, movement of the actuation tab through the second distance may actuate the spring-loaded release mechanism to separate the clamping cap from the implant housing. For example, movement of the actuation tab through the second distance may release a catch on the spring-loaded release mechanism.

In examples, the implant delivery device may further comprise an implant deployment member comprising a pusher extending from adjacent to the handle into the lumen of the first needle. The implant deployment member may be slidable relative to the first needle to push the implant housing out of the first needle. The implant deployment member may be hollow and an implant housing retaining member may extend through the implant deployment member. The implant deployment member may include a tab for an operator to actuate the implant deployment member. An implant housing retaining member may be releasable from the tab of the implant deployment member.

In examples, the implant delivery device may further comprise a medical implant comprising an implant housing disposed in the lumen of the first needle and an electrode lead extending from the implant housing and carried by the second needle. The electrode lead may be carried in a lumen of the second needle, or may be attached to the second needle at an attachment point at or near the distal end and extend along a side of the second needle.

The combined implant delivery device and medical implant may be packaged for single use.

In examples, the medical implant is an implantable neurostimulation device. The medical implant may be configured for stimulating a nerve of the patient, for example the greater occipital nerve. In other examples, the medical implant may be a non-stimulation implant, for example a diagnostic implant. The diagnostic implant may comprise an implant housing and an antenna for wireless communication with an external device. The stimulation implant may be implanted such that the implant housing is positioned at a first depth by the first needle, and the antenna is implanted at substantially the same depth by the second needle, for example at a substantially even distance from the outer surface of the skin. To achieve this the first and second needles can be pushed into the patient substantially parallel with the skin.

### BRIEF DESCRIPTION OF THE DRAWINGS

Embodiments of the invention are further described hereinafter with reference to the accompanying drawings, in which:
FIG. 1 shows a medical implant;
FIG. 2 show the medical implant in position in a patient, particularly in a subcutaneous position;
FIG. 3 shows a schematic cross-section of a part of a delivery device for implanting the medical implant into the patient, particularly a first needle and a second needle of the delivery device;
FIG. 4 shows a schematic cross-section of the delivery device, including a handle, in a configuration before implanting the medical implant;
FIG. 5 shows a schematic cross-section of the delivery device after the electrode lead of the medical implant has been released;
FIG. 6 shows a schematic cross-section of the delivery device after the medical implant has been released;
FIG. 7 shows a perspective view of an end of the first needle and the second needle, and a medical implant retained by the first and second needles;
FIG. 8A shows the second needle;
FIG. 8B shows the first needle;
FIG. 9 shows an example delivery device for implanting the medical implant into the patient;
FIG. 10 shows the second needle of the delivery device of FIG. 9, with an actuation tab;
FIG. 11 shows an alternative view of the delivery device of FIG. 9;
FIG. 12 shows a locking mechanism for the second needle and actuation tab of the delivery device of FIG. 9;
FIGS. 13A and 13B show an example of a retaining mechanism for releasably retaining the implant housing of the medical implant in the first needle;
FIGS. 14A to 14C show a further example of a retaining mechanism for releasably retaining the implant housing of the medical implant in the first needle;
FIGS. 15A to 15C show a further example of a retaining mechanism for releasably retaining the implant housing of the medical implant in the first needle;
FIG. 16 illustrates an example implant delivery device in which the second needle is initially in a retracted position;
FIGS. 17A to 17E illustrate operation of the delivery device to implant the medical implant in a patient;
FIGS. 18A and 18B show schematic cross-sections of a part of a further example delivery device for implanting the medical implant into the patient, wherein the actuation tab is moved towards the handle during use;
FIG. 19 shows a perspective view of the example delivery device of FIGS. 18A and 18B;
FIG. 20 shows a partial cross-section of the delivery device of FIG. 19, showing the rack and pinion mechanism;
FIGS. 21A and 21B show schematic cross-sections of a part of a further example delivery device for implanting the medical implant into the patient, wherein the second needle is initially in a retracted position;
FIGS. 22A and 22B show a release mechanism of the example delivery devices of FIGS. 18A to 21B for releasing the implant housing from the first needed during use;
FIGS. 23A to 23E illustrate operation of the delivery device of FIGS. 18A to 22B to implant the medical implant in a patient;
FIG. 24 illustrates an alternative example second needle having an attachment point for attachment of the electrode lead;
FIG. 25 illustrates an alternative example first needle having a sharp protrusion; and
FIG. 26 illustrates an alternative example first needle with a window corresponding with the implant housing within the first needle.

### DETAILED DESCRIPTION

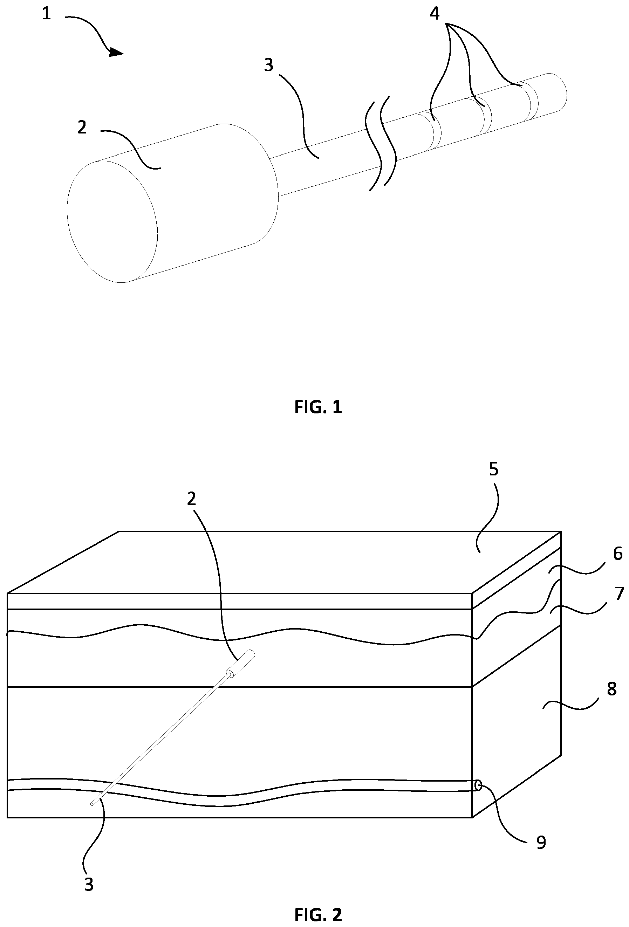

FIGS. 1 and 2 illustrate the medical implant 1 and use of the medical implant 1 in a patient. As described further hereinafter, the medical implant 1 is implantable in a patient and operable to sense and/or stimulate a nerve. In some examples, the medical implant 1 is implantable to sense and/or stimulate the greater occipital nerve, although the same or similar implant may be implantable to sense and/or stimulate other nerves, particularly other peripheral nerves of the peripheral nervous system. In examples, the medical implant 1 may be implantable to sense and/or stimulate the tibial nerve, the sacral nerve (e.g., to treat urinary incontinence) or the vagus nerve (e.g., to regulate pancreatic secretion).

As shown in FIG. 1, the medical implant 1 comprises an implant housing 2 and an elongate electrode lead 3. The implant housing 2 may house electronics components of the medical implant 1, including for example a printed circuit board, a receiver/transmitter, a wireless power receiver, and/or sensor electronics. In examples, the implant housing is hermetically sealed.

The electrode lead 3 extends from the implant housing 2 and is flexible. The electrode lead 3 includes at least one electrode 4, in some examples multiple electrodes 4 spaced along the length of the electrode lead 3. The electrodes 4 are connected to the electronics within the implant housing 2.

In examples, the implant housing 2 may have a diameter of between about 0.5 millimetres and about 5 millimetres, for example between about 1 millimetre and about 3 millimetres. The implant housing 2 may have a length of up to about 10 millimetres, for example up to about 5 millimetres. In examples, the electrode lead 3 may have a diameter of between about 0.3 millimetres to about 1.5 millimetres, for example between about 0.5 millimetres and 1.3 millimetres. The electrode lead 3 may have a length of up to about 100 millimetres, for example up to about 50 millimetres, for example about 50 millimetres. However, it will be appreciated that the dimensions of the implant housing 2 would correspond to the size of the electronics housed within the implant housing 2, and the length of the electrode lead 3 would correspond to the anatomy surrounding the targeted nerve, so a shorter or longer electrode lead 3 may be appropriate depending on the depth of the nerve within the muscle tissue.

As shown in FIG. 2, once implanted in a patient the medical implant 1 is positioned below the surface of the skin, in particular below the epidermis 5. The implant housing 2 may be positioned in the dermis 6 or in the subcutaneous tissue 7. Positioning the implant housing 2 in the subcutaneous tissue 7 may be beneficial to cause less damage and/or irritation to the patient.

As illustrated, the electrode lead 3 extends from the implant housing 2, through the underlying tissue, in particular muscle 8, to a position proximal to the target nerve 9. The electrode lead 3 is positioned such that the electrodes (4, see FIG. 1) are in contact with or proximal to the nerve 9, and so the electrodes 4 can be used to sense and/or stimulate the nerve 9.

An external device (not shown) can wirelessly power, and wirelessly communicate with, the medical implant 1, in particular the electronics in the implant housing 2. The external device may be positioned on the skin proximal to the medical implant 1. The external device may be adhered to the skin proximal to the medical implant 1.

In examples, the medical implant 1 is a neurostimulation implant that is implanted to target the greater occipital nerve of a patient, and the electrodes 4 provided with an electrical signal, such as a current, to stimulate the greater occipital nerve. In examples, the electrical signal may be a voltage regulated stimulation. Such stimulation can provide relief for chronic pain, for example occipital neuralgia, intractable migraine, and/or other therapeutic benefits.

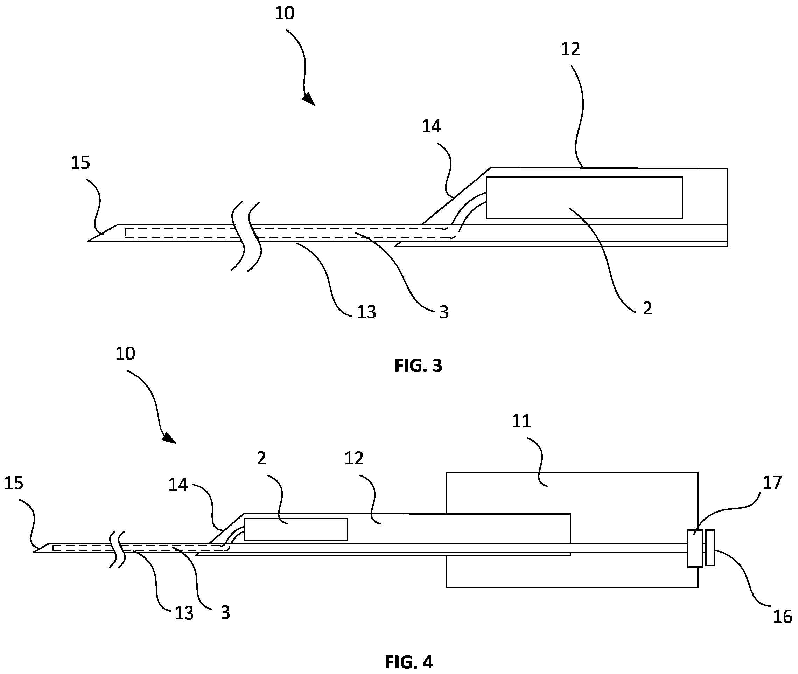

FIGS. 3 to 22E illustrate examples of implant delivery devices 10 for implanting the medical implant 1 illustrated in FIG. 1 in the location illustrated in FIG. 2.

FIGS. 3 to 16D illustrate a first example implant delivery device 10. As shown in FIG. 3, the implant delivery device 10 includes a first needle 12 and a second needle 13. The first and second needles 12,13 are parallel and both extend in a longitudinal direction. The second needle 13 extends further than the first needle 12. In particular, the first needle 12 has a tip 14, such as a bevel tip, and the second needle 13 extends past the tip 14 of the second needle. The second needle 13 also has a tip 15, in particular a bevel tip. The second needle 13 has a higher gauge than the first needle 12, and in particular the second needle 13 has a smaller diameter than the first needle 12.

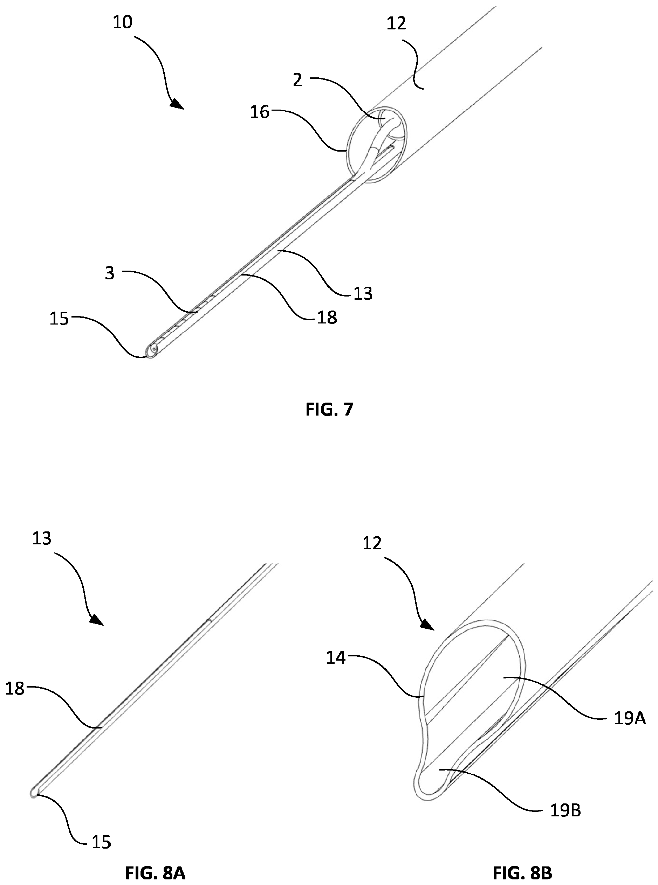

During use, the implant housing 2 is received in the first needle 12, in particular in a lumen of the first needle 12. During use, the electrode lead 3 is received in the second needle 13, in particular in a lumen of the second needle 13. The electrode lead 3 extends along a substantial part of the second needle 13 towards the tip 15, as illustrated. A part of the electrode lead 3 adjacent to the implant housing 2 extends through an opening in the second needle 13, as described further with reference to FIG. 7. Accordingly, during use the medical implant 1 is housed within the first and second needles 12, 13 of the delivery device 10.

As shown in FIG. 3, the first needle 12 and the second needle 13 are axially offset. In particular, a central axis of the first needle 12 is offset from a central axis of the second needle 13. In the illustrated example the second needle 13 extends into the first needle 12 (in particular into the lumen of the first needle), such that the second needle 13 is partly accommodated within the first needle 12 alongside the implant housing 2.

Referring to FIG. 7, the second needle 13 includes a slot 18 to permit a part of the electrode lead 3 to extend out of the second needle 13 and connect to the implant housing 2, as shown in FIG. 3. The slot 18 may extend to the tip 15 of the second needle 13, as described further hereinafter.

In some examples the second needle 13 may comprise a sheath, having an opening extending entirely or substantially along one side.

As described in further detail hereinafter, during use the first needle 12 and the second needle 13 both penetrate the patient's skin to simultaneously position the implant housing 2 at a first depth and the electrode lead 3 at a second depth within the patient. The implant delivery device 10 then releases and deploys the medical implant 1 to leave the medical implant 1 in the position illustrated in FIG. 2. The tips 14, 15 of the first and second needles 12, 13, respectively, are sharp tips adapted to pierce the patient's skin and penetrate the tissue to position the first and second needles 12, 13 at the appropriate depth. The tips 14, 15 may be bevel tips, as would be known to the skilled person.

In examples, the first needle 12 may have a gauge of between 6 gauge and 15 gauge, for example 10 gauge. In examples, the second needle 13 may have gauge of between 15 gauge and 25 gauge, for example 20 gauge.

In various examples described hereinafter, the second needle 13 is retractable relative to the first needle 12, to deploy the electrode lead 3. The slot 18 (see FIG. 7) along the second needle 13 permits deployment of the electrode lead 3.

In examples, the implant housing 2 is releasably attached to the first needle 12 (or another part of the implant delivery device 10) and is released prior to deployment.

In examples, the implant housing 2 may be deployed from the first needle 12 simply by pulling the implant delivery device away from the patient and relying on friction between the electrode lead 3 and the patient's tissue to hold the medical implant in place and pull the implant housing 2 from the first needle 12. In other examples, the implant delivery device 10 may include a deployment member adapted to push the implant housing 2 out of the first needle 12 to deploy the implant housing 2 at the appropriate anatomical site.

FIGS. 4 to 6 show additional parts of the example implant delivery device 10 and also illustrate operation of the implant delivery device 10 to deploy the medical implant 1 into the patient.

As illustrated, the example implant delivery device 10 further includes a handle 11. The handle 11 is adapted to be held by an operator. The first needle 12 is fixed to the handle 11.

The second needle 13 extends through the first needle 12 and through the handle 11. An actuation tab 16 is provided on an end of the second needle 13 opposite to the tip 15. In particular, the actuation tab 16 may be a gripping handle or similar for the operator to grip.

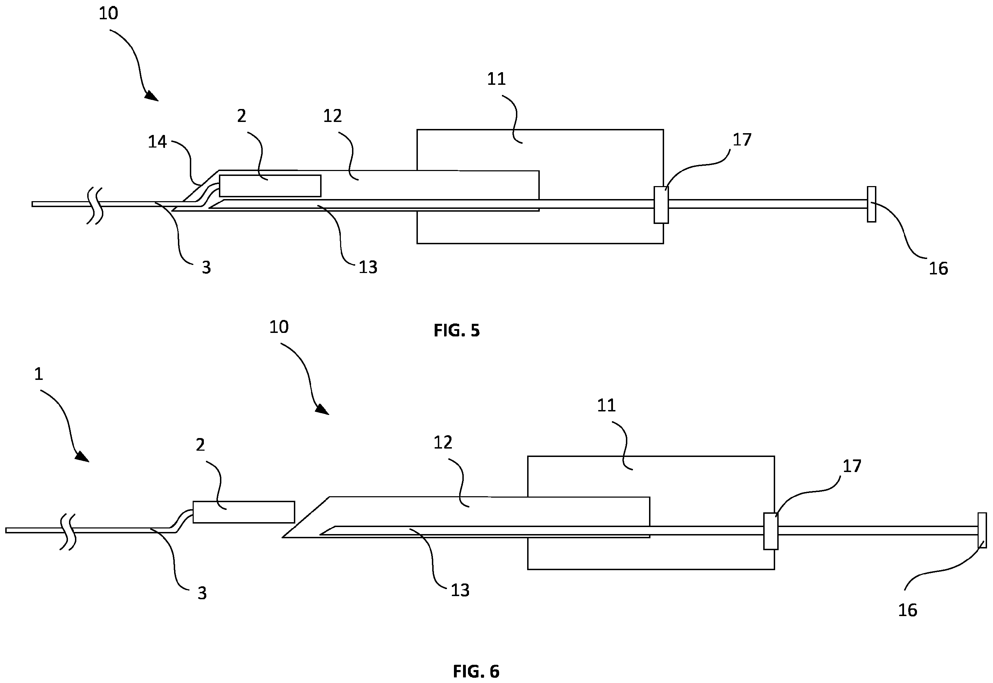

The second needle 13 is retractable relative to the first needle 12. In particular, the second needle 13 can slide through the first needle 12 and handle 11, from the position shown in FIG. 4 and the position shown in FIG. 5. In this example, the second needle 13 is retractable by pulling the actuation tab 16 in a direction away from the patient. Retracting the second needle 13 in this way deploys the electrode lead 3. The slot 18 (see FIG. 7) along the second needle 13 provides for the part of the electrode lead 3 that connects to the implant housing 2. The slot 18 extends to the tip 15 of the second needle 13.

A locking device 17 is provided to lock the second needle 13 to the handle 11 and/or first needle 12. As shown, the locking device 17 may be provided at or near the actuation tab 16, and in examples locks the actuation tab 16 to the handle 11. The locking device 17 locks the second needle 13 in the extended position shown in FIG. 4.

In the position shown in FIG. 4 the operator can insert the implant delivery device 10 into the patient. The position of the second needle 13 is locked relative to the handle 11, so the operator can push the implant delivery device 10 into the patient by the handle 11.

Referring to FIGS. 4 and 2, as the implant delivery device 10 is pushed into the patient the second needle 13 first penetrates the skin 5, and as the implant delivery device 10 is pushed further the first needle 12 then penetrates the skin 5. The first and second needles 12, 13 are thereby positioned at the appropriate depths in the patient, and the implant housing 2 and electrode lead 3 are also simultaneously positioned at the appropriate depths.

In examples, the operator may use an ultrasound imaging device to monitor the positions of the first needle, and in particular the second needle 13, to guide the second needle 13 towards the target nerve 9.

Once the implant delivery device 10 is in position, with the tip 15 of the second needle 13 (and the electrode lead 3 within the second needle 13) being positioned adjacent to the nerve, and the tip 14 of the first needle 12 (and the implant housing 2 within the first needle 12) being positioned in the subcutaneous tissue, the second needle 13 can be retracted to the position shown in FIG. 5.

The second needle 13 is retracted by unlocking the locking mechanism 17 and pulling on the actuation tab 16 relative to the handle 11 to slide the second needle 13 to the retracted position shown in FIG. 5. The actuation tab 16 is pulled in a direction away from the patient. During retraction of the second needle 13 the handle 11 and first needle 12 remain stationary. As shown, once the second needle 13 is retracted the electrode lead 3 is deployed and exposed to the surrounding tissue (and nerve).

Next, as shown in FIG. 6, the implant delivery device 10 is withdrawn from the patient and the implant housing 2 is deployed from the first needle 12. In this example the friction between the electrode lead 3 and the tissue (see muscle 8 in FIG. 2) is enough to hold the medical implant 1 in place as the implant delivery device 10 is withdrawn, thereby deploying the implant housing 2 out of the first needle 12 as the implant delivery device 10 is withdrawn from the patient.

In other examples detailed hereinafter the implant delivery device 10 may include a deployment member configured to push the implant housing 2 out of the first needle 12. In some examples detailed hereinafter, the implant delivery device 10 may have a retaining member arranged to releasably attach the implant housing 2 to the first needle 12 and/or handle 11, and the retaining member can release the implant housing 2 after the second needle 13 is retracted and before the first needle 12 is removed from the patient.

FIGS. 7, 8A and 8B illustrate the first needle 12 and the second needle 13. As shown, the electrode lead 3 is received in the second needle 13, and the second needle 13 includes a slot 18 for connecting to the implant housing 2 in the first needle 12. The slot 18 extends partially along the second needle 13 from the tip 15. The slot 18 may extend the full length of the second needle 13. The second needle 13 may be in the form of a sheath. The first needle 12 receives the implant housing 2.

The first needle 12 includes a bevel tip 14, and the second needle includes a bevel tip 15. The bevel tips 14, 15 are sharp for piercing a patient's skin and penetrating the tissue during use.

As shown in FIG. 7, the second needle 13 extends through the lumen of the first needle 12. In particular, as shown in FIG. 8B the first needle 12 includes a primary portion 19A and a secondary portion 19B. The primary and secondary portions 19A, 19B are merged such that the first needle 12 has a single lumen. The primary and secondary portions 19A, 19B are shaped to define the two distinct portions 19A, 19B.

The primary portion 19A is shaped to receive the implant housing 2. In particular, the primary portion 19A is sized to receive the implant housing 2 and has a substantially circular cross-section that retains the implant housing 2 in axial alignment within the primary portion 19A.

The secondary portion 19B is shaped to receive the second needle 13. In particular, the secondary portion 19B is sized to receive the second needle 13 and has a substantially circular cross-section that retains the second needle 13 in axial alignment within the secondary portion 19B.

In examples, the secondary portion 19B and the primary portion 19A each have a substantially circular cross-section, and the cross-sections at least partially overlap. In such an example, the implant housing may be pushed over to one side of the primary portion 19A by the presence of the second needle 13 in the secondary portion 19B.

Accordingly, the first needle 12 is shaped to receive the implant housing 2 and the second needle 13, and to permit the second needle 13 to slide towards the retracted position. The position of the second needle 13 within the lumen of the first needle 1 beneficially means that there is only one puncture wound formed in the patient's skin, as the first needle 12 will enlarge the puncture wound formed by the second needle 13 during use.

In alternative examples the second needle 13 does not pass into, or through, the lumen of the first needle 12. Instead, the second needle 13 can extend through another part of the handle 11.

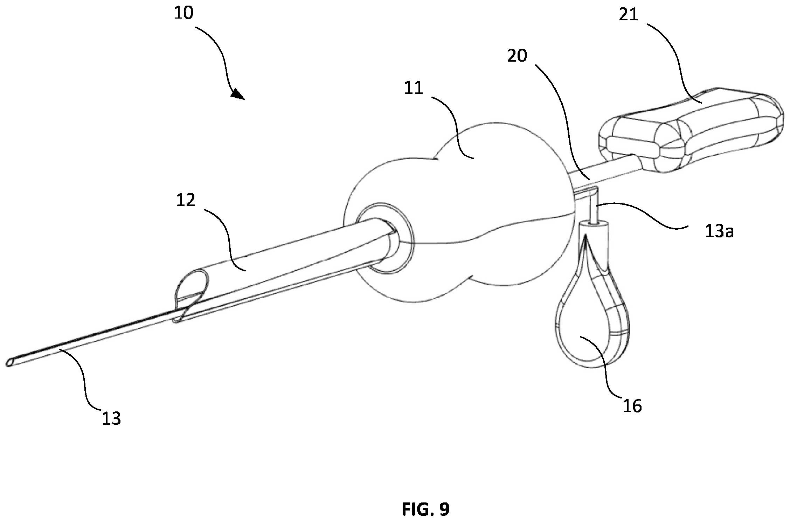

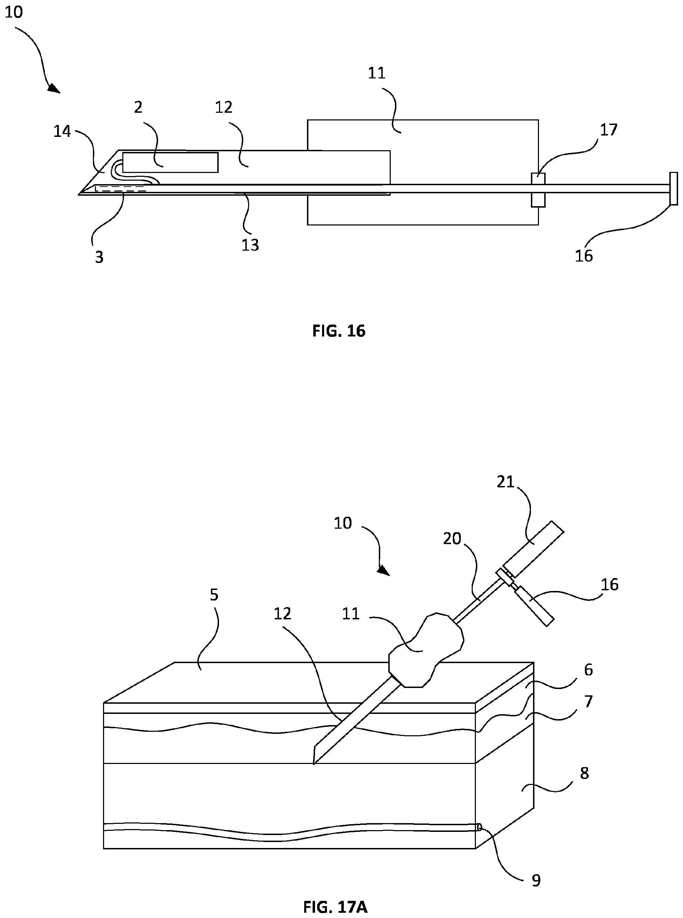

FIGS. 9 to 11 illustrate an example implant delivery device 10. As illustrated, the implant delivery device 10 includes a handle 11, a first needle 12 and a second needle 13 in the configuration as described above with reference to FIGS. 3 to 8B. In particular, the first needle 12 is fixed to the handle 11 and the second needle 13 is retractable from an extended position (shown in FIG. 9) where it extends past the end of the first needle 12, and a retracted position (see FIG. 16D) where the second needle 13 is retracted towards the handle 11 to release the electrode lead (3, see FIG. 3).

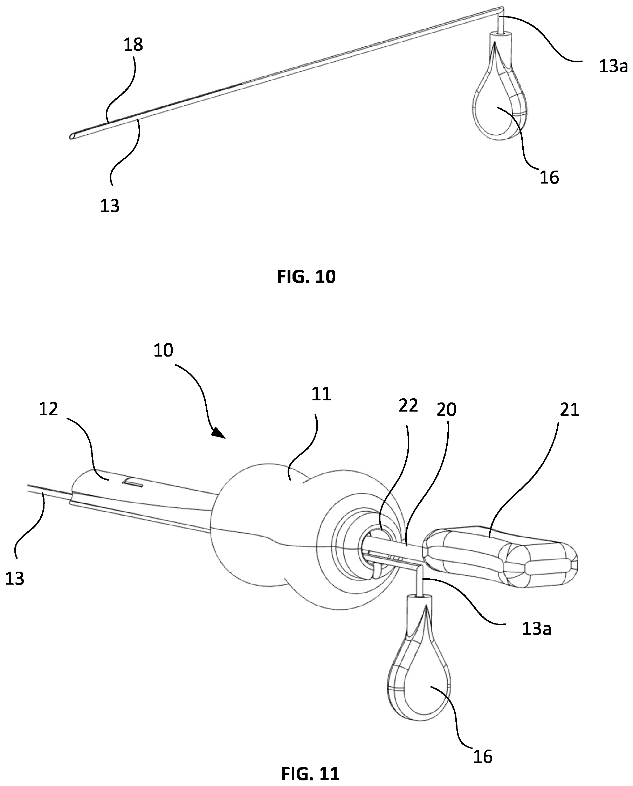

As shown in FIGS. 9 and 10, the second needle 13 extends through the handle 11 and a portion 13a of the second needle 13 is attached to the actuation tab 16.

Also shown in FIG. 9 is an implant deployment member 20, which includes a gripping tab 21. As explained further hereinafter, the implant deployment member 20 may be in the form of a tube or plunger arranged to slide into the first needle 12 and push the implant housing 2 out of the first needle 12.

As shown in FIG. 11, the handle 11 includes an opening 22 in which the first needle 12 is mounted, and through which the second needle 13 and the implant deployment member 20 pass.

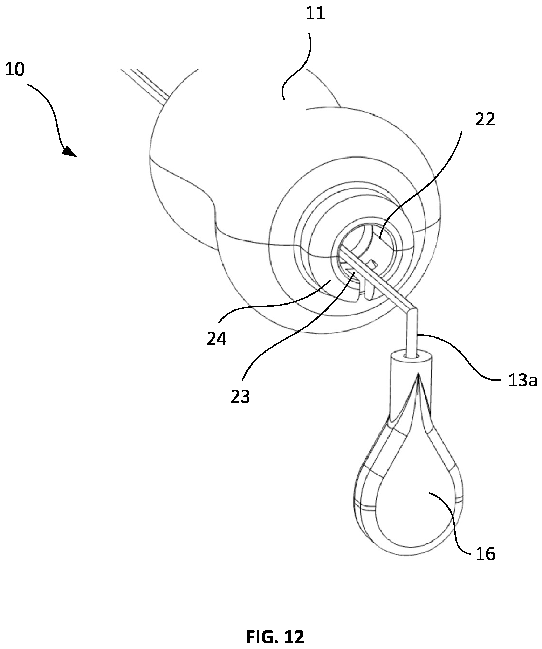

FIG. 12 shows an example of the locking mechanism 17 described with reference to FIGS. 4 to 6. The locking mechanism is arranged to releasably lock the position of the second needle 13 relative to the handle 11 and first needle 12. In this example, the locking mechanism comprises a locking slot 23 formed in a locking ring 24 on the handle 11. The locking ring 24 may be an integral part of the handle 11 or attached to the handle 11. The locking slot 23 may be an L-slot or a J-slot shaped to receive a part of the second needle 13, in particular the portion 13a of the second needle 13 that the actuation tab 16 is attached to. The portion 13a can enter the slot and rotation of the second needle 13 (by the actuation tab 16) moves the portion 13a into an end of the locking slot 23 that restrains axial movement of the second needle 13. Accordingly, the second needle 13 can be moved between a locked position and an unlocked position by rotating the second needle 13, in particular the actuation tab 16, to move the portion 13a into and out of the locking slot 23.

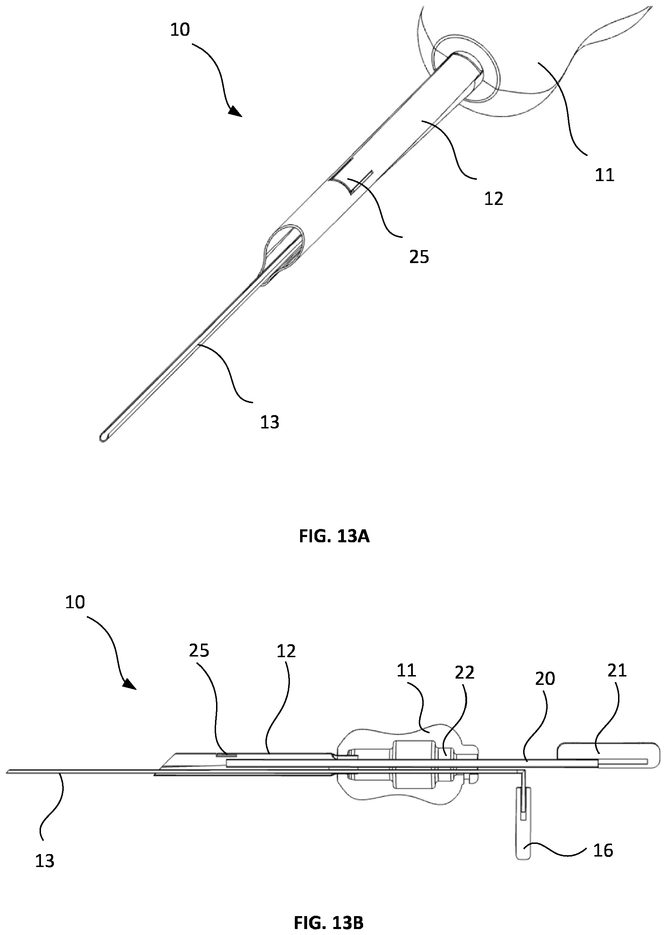

FIGS. 13A and 13B illustrate an example of a retaining member configured to releasably secure the implant housing 2 in the first needle 12. As shown, in this example the first needle 12 comprises a retaining member in the form of a retaining tab 25 arranged to contact the implant housing (2, see FIG. 7) and retain the implant housing in the first needle 12. The retaining tab 25 is formed by a U-shaped cut in the first needle 12 that forms the retaining tab 25, which is partially bent inwards to narrow the internal diameter of the first needle 12 at the location of the retaining tab 25.

In some examples, the implant housing 2 may comprise a recess arranged to correspond with the retaining tab 25. In other examples, the retaining tab 25 contacts a side wall of the implant housing 2.

The retaining tab 25 may be configured such that the implant housing 2 can be pulled from the first needle 12 with a relatively low force to deploy the implant housing 2 from the first needle 12. Accordingly, friction between the electrode lead 3 and tissue of the patient may provide sufficient holding force for the retaining force of the retaining tab 25 to be overcome by pulling the implant delivery device 10 from the patient after retracting the second needle 13, as described above.

In another example, the implant delivery device 10 further comprises an implant deployment member 20 as shown in FIG. 13B. The implant deployment member 20 extends through the opening 22 in the handle and into the first needle 12. From here, the implant deployment member 20 can function as a plunger to push the implant housing 2 out of the first needle 12. The implant deployment member 20 is provided with a gripping tab 21 for actuation of the implant deployment member 20.

In examples, the implant deployment member 20 may comprise a locking tab arranged to prevent axial movement of the implant deployment member 20 towards the implant housing 2 until the locking tab is disengaged, for example by rotation of the implant deployment member 20 or gripping tab 21.

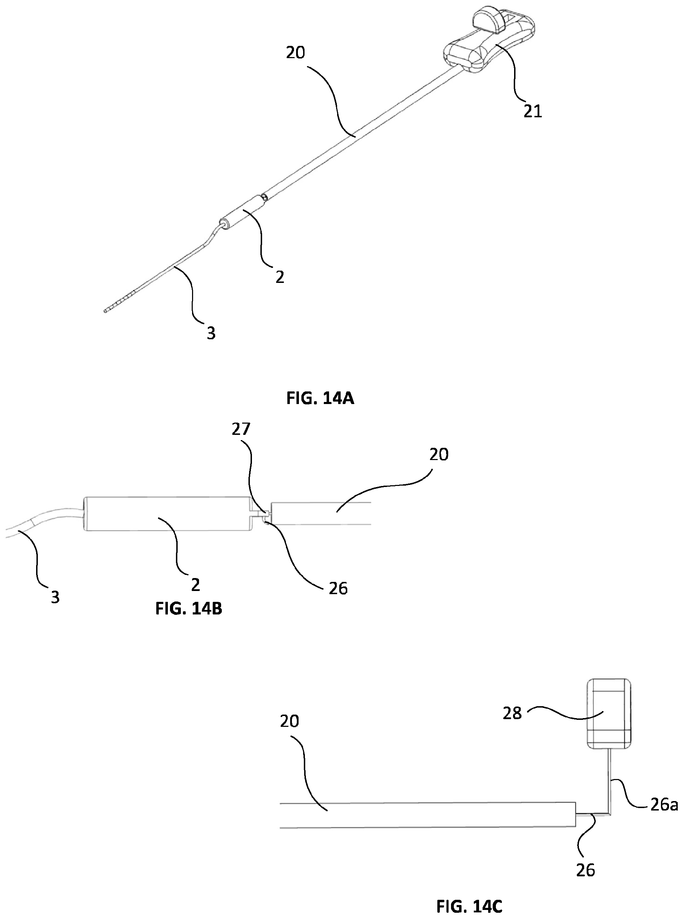

FIGS. 14A to 14C illustrate another example of a retaining member configured to releasably secure the implant housing 2 in the first needle 12. In this example, the retaining member comprises a shape memory member 26 that extends through the handle 11, and in particular through the implant deployment member 20, between the gripping tab 21 and the implant housing 2.

As shown in FIG. 14B, the implant housing 2 comprises an attachment point 27, for example a hole or hook, that an end of the shape memory member 26 hooks onto to hold the implant housing 2 in the first needle 12.

As shown in FIG. 14C an opposite end 26a of the shape memory member 26 to the hook 27 is attached to an actuation tab 28 that is slidably mounted to the gripping tab 21. Accordingly, moving the actuation tab 28 relative to the gripping tab 21 pulls on shape memory member 26. Pulling the actuation tab 28 can thereby deform the hook at the end of the shape memory member 26 and disengage it from the attachment point 27, thereby releasing the implant housing 2.

In examples the shape memory member 26 comprises a shape memory polymer or a shape memory alloy such as a Nitinol bar. In other examples the shaper memory member 26 may comprise a bi-metallic strip, spring wire, or piano wire. The shape memory member 26 has a hooked end that engages the attachment point 27 on the implant housing 2. The hooked end can be formed as shape memory and act to prevent the implant housing 2 from moving out of the first needle 12 until the actuation tab 28 has been pulled to disengage the hooked end from the attachment point 27.

The actuation tab 28 can be releasably locked relative to the gripping tab 21 to prevent unintentional release of the implant housing 2 from the first needle 12.

Once the shape memory member 26 has been disengaged from the attachment point 27 the implant housing 2 may be pulled out of the first needle 12 by friction between the electrode lead 3 and the tissue of the patient, and/or an implant deployment member 20 may act as a plunger as described previously.

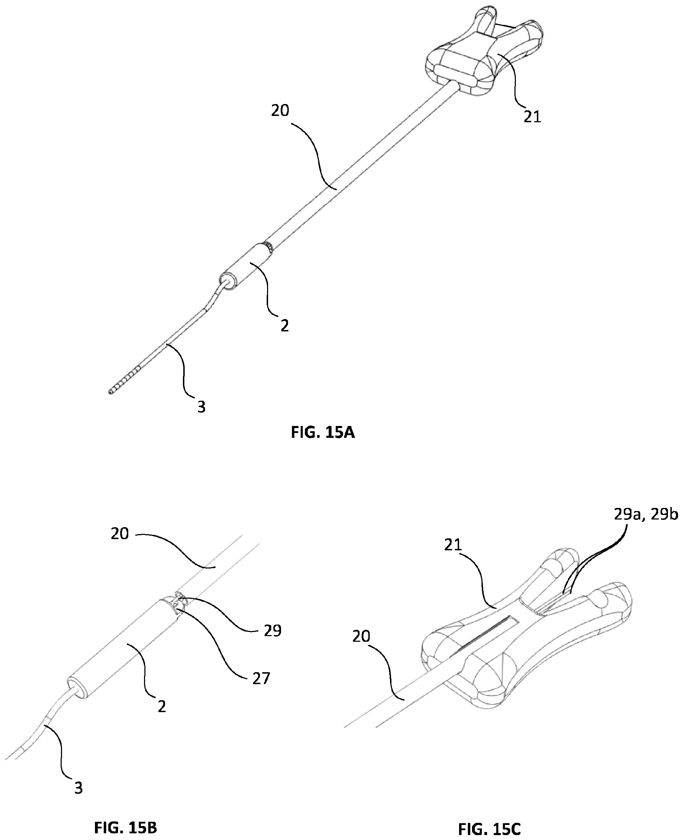

FIGS. 15A to 15C illustrate another example of a retaining member configured to releasably secure the implant housing 2 in the first needle 12. In this example the retaining member comprises a thread 29 extending from the gripping tab 21 to the implant housing 2 in the first needle 12. The thread 29 is looped through the attachment point 27 on the implant housing 2 and both ends 29a, 29b are secured at the gripping tab 21. For example, the ends 29a, 29b may be tied to each other, or to a part of the gripping tab 21. Preferably, the ends 29a, 29b are secured to the gripping tab 21 such that the ends 29a, 29b can be readily released or such that a part of the thread 29 can be easily accessed to be cut.

Accordingly, the thread 29 acts to hold the implant housing 2 in the first needle 12 until the thread 29 is cut or untied, allowing the thread 29 to be removed.

Once the implant housing 2 has been released the implant housing 2 may be pulled out of the first needle 12 by friction between the electrode lead 3 and the tissue of the patient, and/or an implant deployment member 20 may act as a plunger as described previously.

The thread may be a Kevlar thread.

In the above-described examples the second needle 13 is initially in an extended position, and the first and second needles 12, 13 can be simultaneously positioned in the patient. FIG. 16 illustrates an alternative example in which the second needle 13 is initially in a retracted position. In this example, as shown, the actuation tab 4 extends from the handle 11. In the retracted position the second needle 13 may be within the first needle 12, as illustrated, or alongside the first needle 12 within the handle 11. The electrode lead 3 is partially within the second needle 13 and looped between the implant housing 2 and the second needle 13.

During use, as described further hereinafter, the first needle 12 is inserted into the patient with the implant delivery device 10 in the configuration shown in FIG. 16, then the actuation tab 16 is pushed towards the handle 11 to extend the second needle 13 beyond the first needle 12 and carry the electrode lead 3 into position. In this position the locking mechanism 17 can be engaged such that the actuation tab 16 and second needle 13 are locked in position.

In some examples, the implant delivery device 10 may be removed from the patient with the second needle 13 in the extended position, and a pusher (as described above) may be provided to urge the medical implant 1 out of the first and second needles 12, 13. In other examples, after being extended the second needle 13 can then be retracted by pulling the actuation tab 16 away from the handle 11 in the manner described above, to release the electrode lead 3.

FIGS. 17A to 17E illustrate a method of operating the implant deployment device 10 to deploy the medical implant 1 into a patient.

In these examples the implant delivery device 10 comprises a handle 11, a first needle 12 with the implant housing 2, and a second needle with the electrode lead 3 as described above. The implant delivery device 10 also includes an actuation tab 16 for retracting the second needle 13, and an implant deployment member 20 for pushing the implant housing 2 out of the first needle 12 as described above.

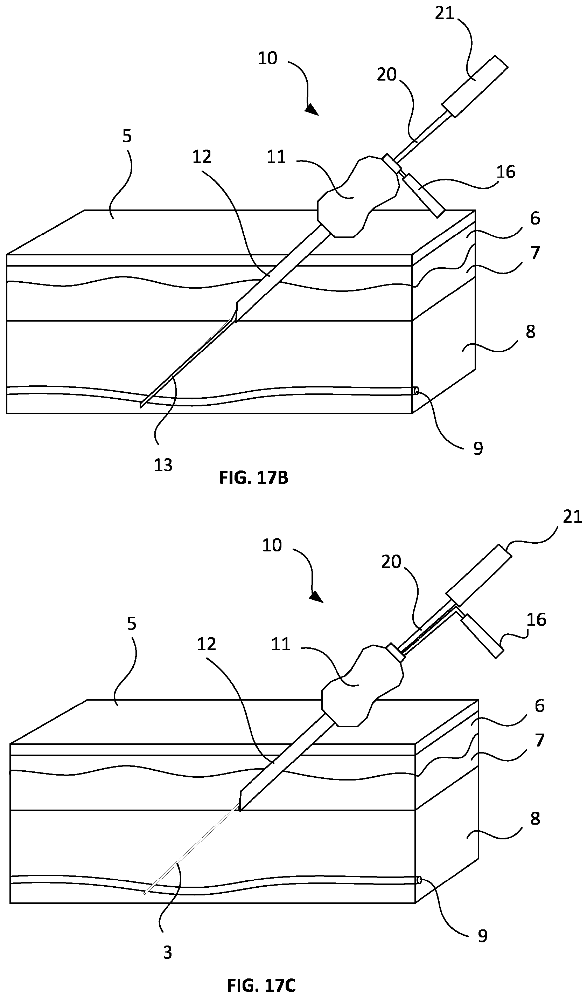

The implant delivery device 10 of FIGS. 17A to 17E may include a retaining member as described in any of FIGS. 13A to 15C. As will become apparent, FIG. 17A is relevant to the example of FIG. 16, in which the second needle 13 is initially in a retracted position, and FIGS. 17B to 17E are relevant to all of the examples of FIGS. 3 to 16.

FIG. 17A shows the implant delivery device 10 in an initial state, with the second needle 13 retracted as described with reference to FIG. 16. In this state an operator pushes the first needle 12 through the patient's skin 5 and into the position for deploying the medical implant 1.

The implant delivery device 10 and medical implant 1 are pre-assembled and packaged as a single-use unit. Accordingly, the operator is provided with an implant delivery device 10 with a medical implant 1 already loaded into the first and second needles 12, 13.

Then, as shown in FIG. 17B, the actuation tab 16 is pushed towards the handle 11 to extend the second needle 13 beyond the first needle 12, to a second depth. The second needle 13 carries the electrode lead 3. The operator may use an ultrasound imaging device to help guide the second needle 13 towards the nerve 9. In this state the first needle 12 has positioned the implant housing 2 at the appropriate depth within the patient's tissue and the second needle 13 has simultaneously positioned the electrode lead 3 at the appropriate depth within the patient's tissue.

FIG. 17B also shows the implant delivery device 10 of FIGS. 3 to 9 in an initial state, with the second needle 13 in an extended position covering the electrode lead (not shown in FIG. 17B). The second needle 13 is locked to the handle 11 by a locking mechanism, for example the locking slot 23 described with reference to FIG. 12.

The implant delivery device 10 and medical implant 1 are pre-assembled and packaged as a single-use unit. Accordingly, the operator is provided with an implant delivery device 10 with a medical implant 1 already loaded into the first and second needles 12, 13.

In this state an operator pushes the first and second needles 12, 13 through the patient's skin 5 and into the position for deploying the medical implant 1. The operator may use an ultrasound imaging device to help guide the second needle 13 towards the nerve 9.

In the appropriate position, shown in FIG. 17B, the second needle 16 is in the muscle tissue 8, proximal to the nerve 9, and the end of first needle 12 that houses the implant housing 2 is positioned in the subcutaneous tissue 7. Both the first and second needles 12, 13 have passed through the epidermis 5 and dermis 6. As the second needle 13 is arranged within the lumen of the first needle 12 there is only a single puncture through the epidermis 5 and dermis 6.

In one example, the second needle 13 may comprise one or more side openings corresponding to the electrodes (4, see FIG. 1) on the electrode lead, which permits the position of the electrode lead to be tested before retracting the second needle 13. If such testing shows that the electrode lead is not in the correct location relative to the nerve 9 then the implant delivery device 10 can be at least partially removed and the second needle 13 can be repositioned.

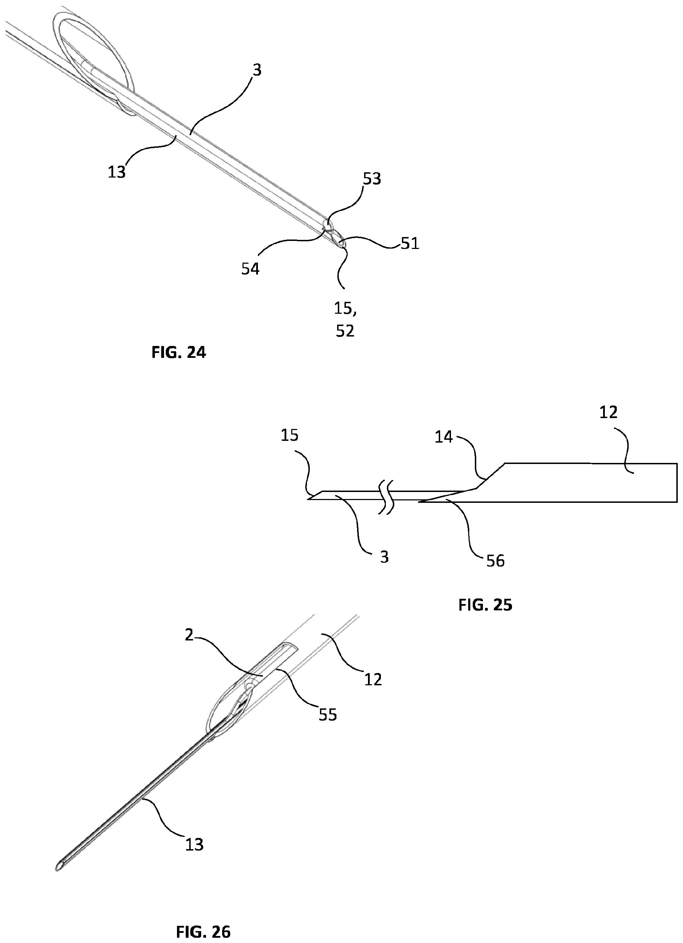

In some examples, as described further with reference to FIG. 26, the first needle 12 may be made from metal, for example stainless steel or aluminium. In such examples the first needle 12 may comprise a non-conductive window, for example formed by a plastic insert or the like, to allow wireless communication between an external device and the implant housing 2 while the implant housing 2 is positioned within the first needle 12. This allows the position of the electrode lead to be tested, and also allows the electronics provided in the implant housing 2 to be tested before the implant is deployed.

As shown in FIG. 17C, next the operator unlocks the second needle 13 (e.g., rotates the second needle 13 to disengage the locking mechanism of FIG. 12), and retracts the actuation tab 16 away from the patient. This moves the second needle 13 into the retracted position within the first needle 12 and releases the electrode lead 3.

In one example, the second needle 13 is only partially retracted in order to expose only an end of the electrode lead 3, where the electrodes (4, see FIG. 1) are located. This partial retraction permits testing of the position of the electrode lead 3 relative to the nerve 9. If such testing shows that the electrode lead is not in the correct location relative to the nerve 9 then the second needle 13 can be pushed back out again to re-sheath the electrode lead 3, and the implant delivery device 10 can be at least partially removed and the second needle 13 can be repositioned.

Once the position of the electrode lead 3 is correct, the second needle 13 is fully retracted to release the electrode lead 3.

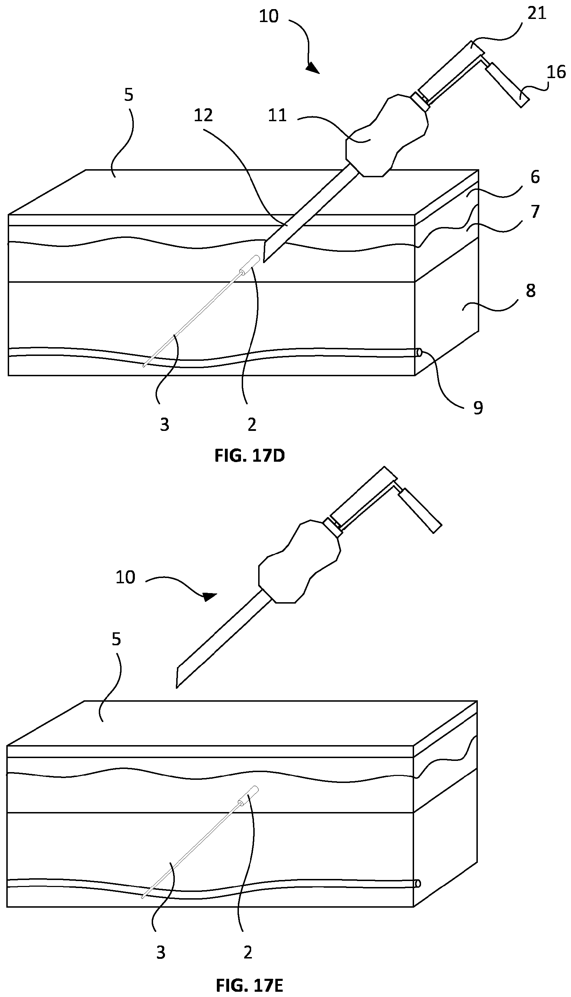

Next, as shown in FIG. 17D, the implant housing 2 is released and deployed from the first needle 12. In particular, if the implant delivery device 10 includes a retaining member as described in any of FIGS. 13A to 15C, then the retaining member is released to release the implant housing 2.

In some examples, friction between the electrode lead 3 and the tissue, in particular the muscle 8, provides a holding force sufficient to pull the implant housing 2 out of the first needle 12 when the implant delivery device 10 is withdrawn from the patient.

In other examples, as illustrated in FIG. 17D, the implant deployment member 20 (and gripping tab 21) are pushed towards the handle 11 to push the implant housing 2 out of the first needle 12. The implant deployment member 20 may be pushed towards the handle 11 while simultaneously moving the handle 11 away from the skin 5 to deploy the implant housing 2 without pulling or pushing on the electrode lead 3.

Accordingly, the medical implant 1 is deployed, as shown in FIG. 17E, and the implant delivery device 10 can be removed from the patient.

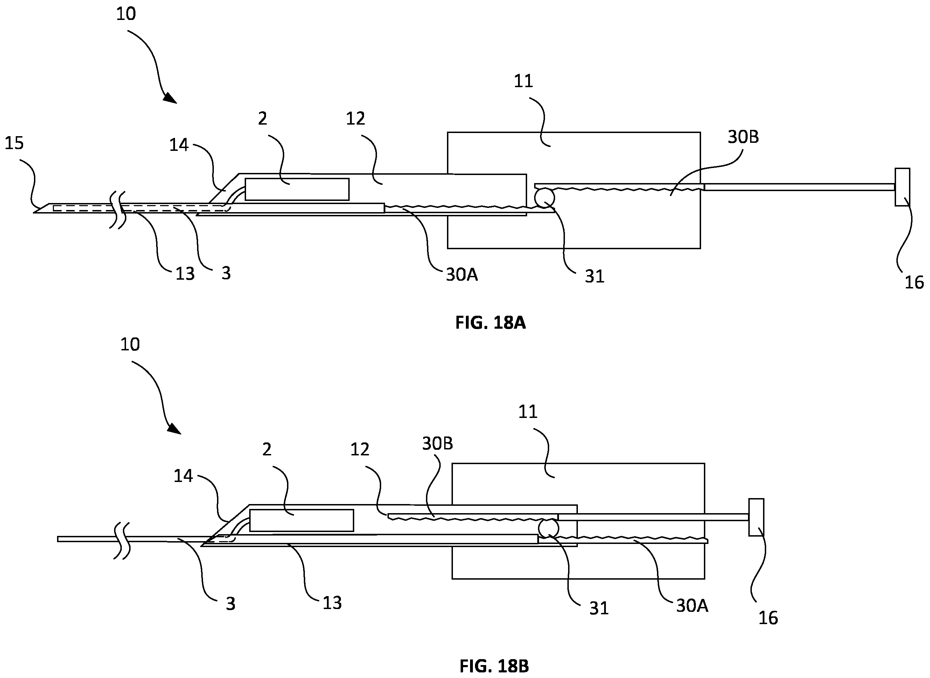

FIGS. 18A to 21B illustrate an alternative example implant delivery device 10. In this example the implant delivery device 10 comprises a handle 11, a first needle 12 holding the implant housing 2 and a second needle 13 holding the electrode lead 3, as previously described. In this example, an actuation tab 16 is arranged to be pushed towards the handle 11 and the patient in order to retract the second needle 13.

In particular, as illustrated, the implant delivery device 10 has a rack and pinion mechanism for translating movement of the actuation tab 16 towards the patient into retraction of the second needle 13 away from the patient. As shown, the rack and pinion mechanism comprises a first rack portion 30A attached to, or formed as part of, the second needle 13. The rack and pinion mechanism also comprises a second rack portion 30B attached to, or formed as part of, the actuation tab 16. A pinion gear 31 is rotatably mounted within the handle 11 and is engaged with both the first and second rack portions 30A, 30B. The pinion gear 31 may be mounted within the first needle 12 or, as illustrated, behind the end of the first needle 12 within the housing 11. Accordingly, as illustrated in FIG. 18B, as the actuation tab 16 is pushed towards the patient the rack and pinion mechanism causes the second needle 13 to be retracted away from the patient to release the electrode lead 3.

Advantageously, pushing the actuation tab 16, rather than pulling the actuation tab 16, may permit one-handed operation of the implant delivery device 10.

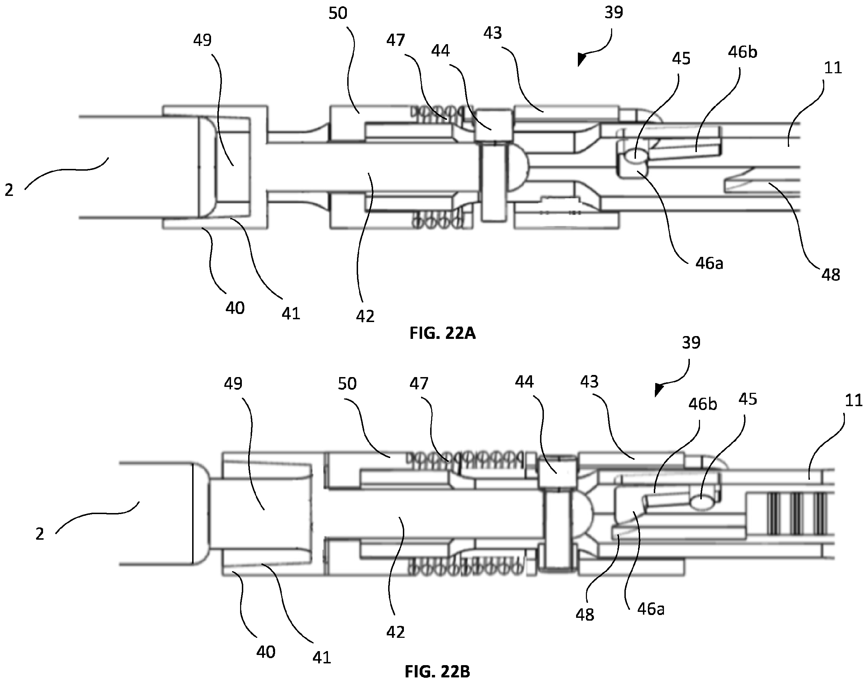

In addition, pushing the actuation tab 16 towards the patient may release the implant housing 2 from the first needle 12, as described in more detail hereinafter with reference to FIGS. 22A and 22B.

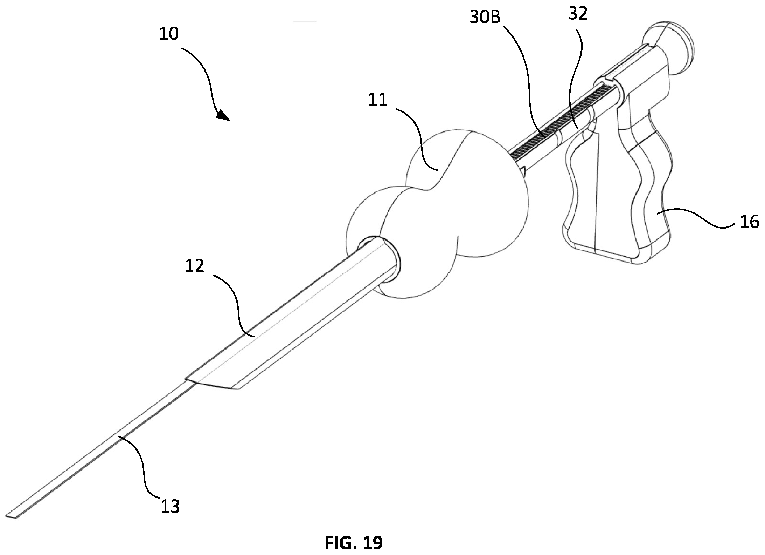

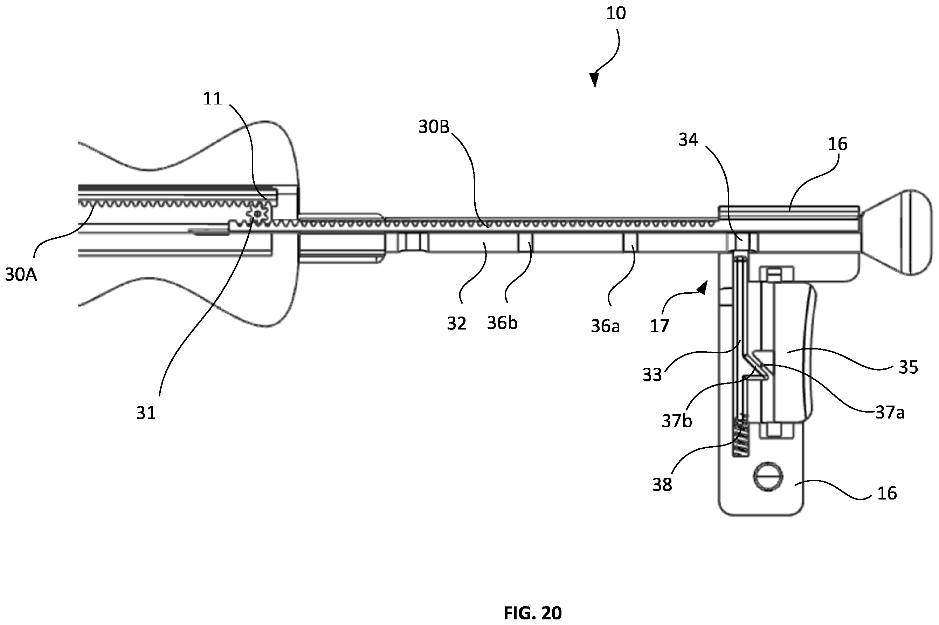

FIGS. 19 to 21B illustrate an example implant delivery device 10 having the push actuation tab 16 and rack and pinion mechanism described with reference to FIGS. 18A and 18B. As illustrated, the implant delivery device 10 is similar to the example of FIG. 9, with a handle 11, first needle 12, and second needle 13. In this example, as more clearly illustrated in FIG. 20, a guide member 32 extends from the handle 11 in the direction away from the patient (oppositely to the needles 12, 13). The guide member 32 is fixed to the handle 11. The actuation tab 16 and second rack portion 30B are slidable along the guide member 32 towards the handle 11 for retraction of the second needle 13 as described above.

As also shown in FIG. 20, a locking mechanism 17 is provided to lock the actuation tab 16 to the guide member 32. The locking mechanism 17 locks the actuation tab 16 (and the second rack portion 30B) to the guide member 32 and therefore also the handle 11 to permit the first and second needles 12, 13 to be inserted into the patient with the first and second needles 12, 13 locked to each other.

Once the first and second needles 12, 13 are positioned in the patient the locking mechanism 17 can be disengaged to release the actuation tab 16 and second rack member 30B to slide along the guide member 32, towards the handle 11, to retract the second needle 13.

As shown in FIG. 20, in this example the locking mechanism 17 comprises a spring-loaded pin 33 mounted to the actuation tab 16 and arranged to engage a corresponding hole 34 on the guide member 32. A spring 38 is arranged to urge the pin 33 into the hole 34. A button 35 can be operated by a user to disengage the pin 33 from the hole 34 and thereby release the actuation tab 16 for movement along the guide member 32. As shown, the button 35 and the pin 33 have opposing angled surfaces 37a, 37b that move the pin 33 out of the hole 34 when the button 35 is depressed. The button 35 is arranged to be depressed towards the patient, in the same direction that the actuation tab 16 is moved to retract the second needle 13. In this way, a user can depress the button 35 and push the actuation tab 16 towards the handle 11 in one movement to retract the second needle 13.

As shown, the guide member 32 may comprise one or more intermediate holes 36a, 36b disposed between the hole 34 and the handle 11 to allow the actuation tab 16 to be locked to the guide member 32 at different locations. In examples, the first intermediate hole 36a may correspond to the position of the actuation tab 16 at which the second needle 13 is withdrawn to release the electrode lead. In examples, as described further hereinafter, the second intermediate hole 36b may correspond to the position of the actuation tab 16 at which the release mechanism has released the implant housing.

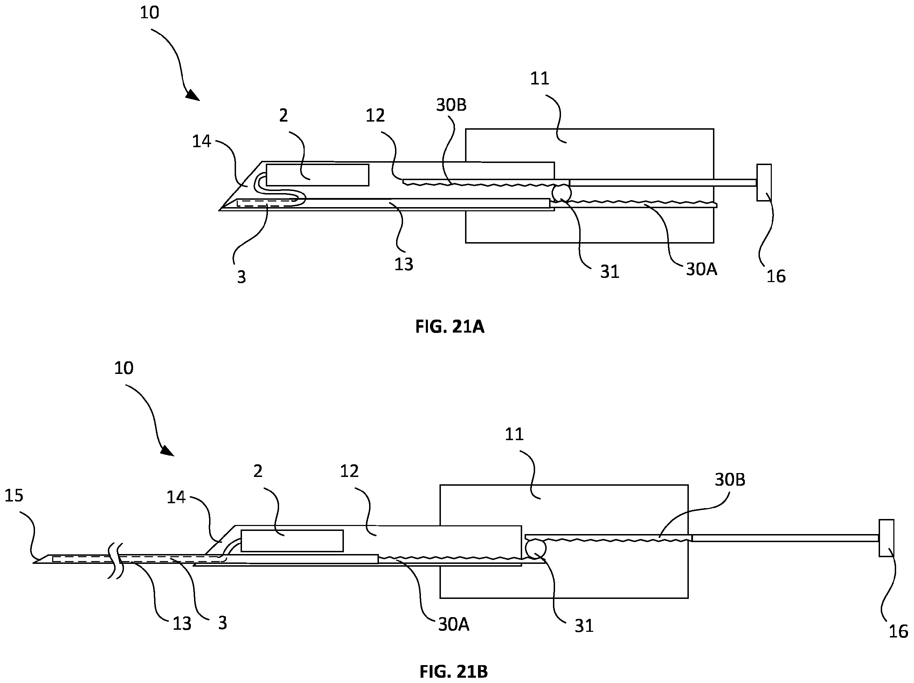

FIGS. 21A and 21B illustrate a similar example implant delivery device 10 to that illustrated in FIGS. 18A to 20. In this example, the implant delivery device 10 comprises a handle 11, a first needle 12 holding the implant housing 2, a second needle 13 holding the electrode lead 3, and an actuation tab 16 arranged to be pushed towards the handle 11 and the patient in order to retract the second needle 13. The implant delivery device 10 has a rack and pinion mechanism to translate movement of the actuation tab 16 into movement of the second needle 13 in the same way as described with reference to FIGS. 18A to 20.

As illustrated in FIG. 21A, in this example the second needle 13 is initially in a retracted position. The retracted position may be within the first needle 12, as illustrated, or alongside the first needle 12 within the handle 11. The electrode lead 3 is partially within the second needle 13 and looped between the implant housing 2 and the second needle 13. In this position the locking mechanism is engaged such that the pin (33, see FIG. 20) is in the first intermediate hole (36a, see FIG. 20).

During use, as described further hereinafter, the first needle 12 is inserted into the patient with the implant delivery device 10 in the configuration shown in FIG. 21A, then the actuation tab 16 is retracted away from the handle 11 to extend the second needle 13 beyond the first needle 12 and carry the electrode lead 3 into position. In this position the locking mechanism is engaged such that the pin (33, see FIG. 20) is in the hole (34, see FIG. 20). The second needle 13 is then retracted by pushing the actuation tab 16 towards the handle 11 in the manner described above, to release the electrode lead 3.

As with the example of FIGS. 18A to 20, the implant delivery device of FIGS. 21A and 21B may have a release mechanism to release the implant housing 2 from the first needle 12.

FIGS. 22A and 22B illustrate an example release mechanism 39 of the implant delivery device 10 of FIGS. 18 to 21B. The release mechanism 39 holds the implant housing 2 within the first needle (not shown in FIGS. 21A and 21B) and is operable to release the implant housing 2 such that the delivery device can be removed leaving the implant 1 in place. FIG. 22A shows the release mechanism 39 engaged with the implant housing 2 and retaining the implant housing 2 within the first needle, and FIG. 22B shows the release mechanism 39 after operation where the implant housing 2 has been released. The release mechanism 39 is housed at least partly within the first needle.

As illustrated, the release mechanism 39 comprises a retaining member, in this example a clamping cap 40. The clamping cap 40 has a tapered recess 41 that can be pushed onto an end of the implant housing 2 to clamp onto the implant housing 2. In the position shown in FIG. 22A, the implant housing 2 is attached to the clamping cap 40, thereby holding the implant housing 2 within the first needle and delivery device.

The clamping cap 40 is movable to a detached position, shown in FIG. 22B, in which the clamping cap 40 is separated from the implant housing 2 to release the implant housing 2 so that the delivery device 10 can be removed leaving the implant in place. As described, the release mechanism 39 is operated by movement of the actuation tab (16, see FIGS. 18 to 21B).

As shown in FIGS. 22A and 22B, the clamping cap 40 further comprises a protrusion 42 extending away from the tapered recess 41 and attached to an actuator member 43 via pin 44. The actuator member 43 is operable to move in the axial direction, away from the implant housing 2, to pull the clamping cap 40 off of the implant housing 2. Movement of the actuator member 43 is constrained by a pin 45 extending from the actuator member 43 through a slot 46 formed in a part of the handle 11 of the delivery device. The slot 46 has a notch 46a in which the pin 45 is positioned before the release mechanism 39 is operated. A spring 47 acts between an anchor 50 that is fixed in the first needle and the actuator member 43 to urge the actuator member 43 in the axial direction, away from the implant housing 2. The notch 46a holds the actuator member 43 in the axial direction against the force of the spring 47. In this position, as shown in FIG. 22A, the release mechanism 39 is spring-loaded.

As the actuation tab (16, see FIG. 20) is moved into the handle 11 a distal end 48 of the actuation tab passes approaches the release mechanism 39 and engages the pin 45. In particular, the distal end 48 of the actuation tab 16 may be formed on the second rack portion 30B illustrated in FIGS. 18A, 18B, 20, 21A and 21B. The distal end 48 is angled so that engagement between the distal end 48 and the pin 45 pushes the pin 45 sideways, out of the notch 46a and into an axially extending portion 46b of the slot 46 as shown in FIG. 22B. This releases the actuator member 43 and clamping member 40 to move away from the implant housing 2 under the force of the spring, and thereby detaches the tapered recess 41 from the implant housing 2 as shown in FIG. 22B.

As shown in FIGS. 22A and 22B, a pusher 49 is provided to prevent the implant housing 2 from moving as the clamping member 40 is moved. In particular, the pusher 49 extends from the anchor 50 and passes through an opening in the clamping member 40 to engage the end of the implant housing 2. Accordingly, when the clamping member 40 is moved by the spring 47 the implant housing 2 is held in position.

As described above, the release mechanism 39 is operable by movement of the actuation tab (16, see FIG. 20) into the handle 11 until the distal end 48 moves the pin 45 out of the slot 46, allowing the spring 47 to move the actuator member 43 and clamping member 40 and release the implant housing 2. It will be appreciated that the release mechanism 39 is operated in this manner at a point after the second needle has been withdrawn.

Accordingly, the embodiments of FIGS. 18A to 20 and 22A and 22B provide an implant delivery device 10 having an actuation tab 16 that is pushed towards the handle 11 during use. In a first stage of the movement of the actuation tab 16 the second needle 13 is retracted to release the electrode lead 3. In a subsequent second stage of the movement of the actuation 16 the release mechanism is operated to release the implant housing 2 from the first needle 12. Accordingly, a user is able to operate the implant delivery device 10 one-handed by pushing the actuation tab 16 towards the handle 11 to initially release the electrode lead 3 and then release the implant housing 2. Once the implant housing 2 has been released the implant delivery device 10 can be removed from the patient.

Similarly, FIGS. 21A to 22B provide an implant delivery device 10 in which initially the actuation tab 16 is pulled away from the handle 11, to extend the second needle 13, and then the actuation tab 16 is pushed towards the handle 11 through first and second stages in the same way as described above for the examples of FIGS. 18A to 20 and 22A and 22B.

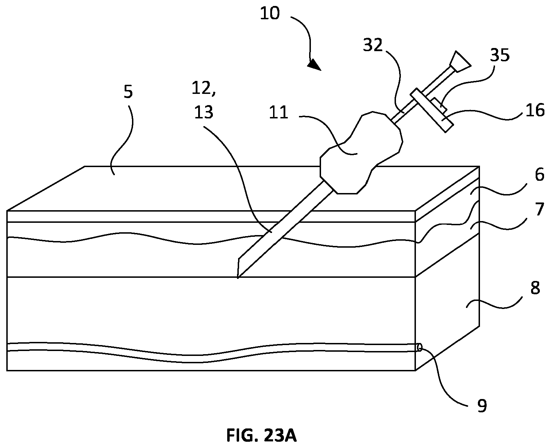

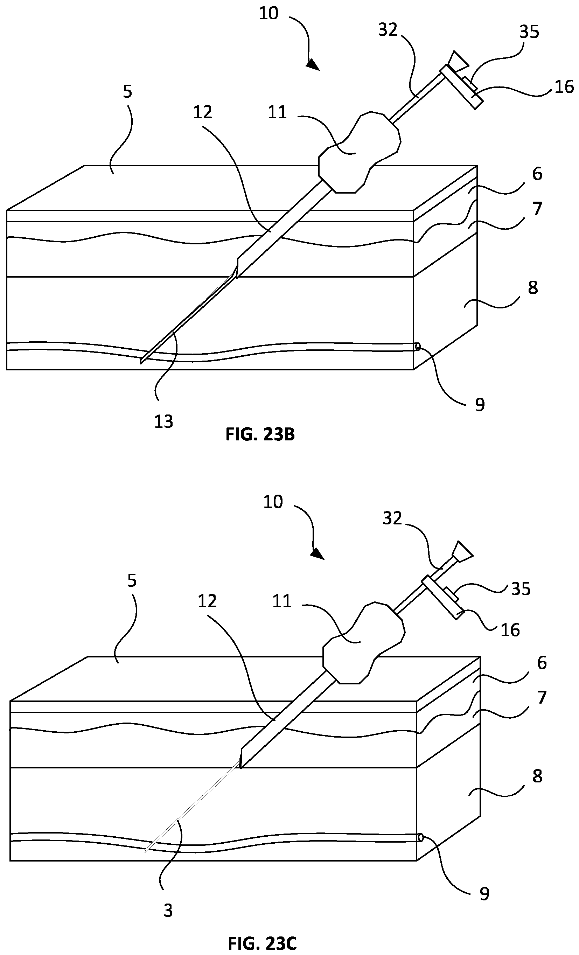

FIGS. 23A to 23E illustrate a method of operating the implant delivery devices 10 of FIGS. 18A to 22B to deploy the medical implant 1 into a patient. As will become apparent, FIG. 23A is relevant to the example of FIGS. 22A and 22B, and FIGS. 23B to 23E are relevant to all of the embodiments of FIGS. 18A to 22B.

FIG. 23A shows the implant delivery device 10 of FIGS. 22A and 22B in the initial state, with the second needle 13 in a retracted position, as also shown in FIG. 22A. In this state an operator pushes the first needle 12 through the patient's skin 5 and into the position for deploying the medical implant 1.

Then, as shown in FIG. 23B, the actuation tab 16 is retracted away from the handle 11 to extend the second needle 13 beyond the first needle 12, to a second depth. The second needle 13 carries the electrode lead 3 as shown in FIG. 22B. In this state the first needle 12 has positioned the implant housing 2 at the appropriate depth within the patient's tissue and the second needle 13 has simultaneously positioned the electrode lead 3 at the appropriate depth within the patient's tissue.

FIG. 23B also shows the initial state of the implant delivery device of FIGS. 18A to 20, with the second needle 13 in an extended position covering the electrode lead (not shown in FIG. 23B). The second needle 13 is locked to the handle 11 by the locking mechanism illustrated in FIG. 20.

In this state an operator pushes the first and second needles 12, 13 through the patient's skin 5 and into the position for deploying the medical implant 1. The operator may use an ultrasound imaging device to help guide the second needle 13 towards the nerve 9.

In the position shown in FIG. 23B, the second needle 16 is in the muscle tissue 8, proximal to the nerve 9, and the end of first needle 12 that houses the implant housing 2 is positioned in the subcutaneous tissue 7. Both the first and second needles 12, 13 have passed through the epidermis 5 and dermis 6. As the second needle 13 is arranged within the lumen of the first needle 12 there is only a single puncture through the epidermis 5 and dermis 6.

In one example, the second needle 13 may comprise one or more side openings corresponding to the electrodes (4, see FIG. 1) on the electrode lead, which permits the position of the electrode lead to be tested before retracting the second needle 13. If such testing shows that the electrode lead is not in the correct location relative to the nerve 9 then the implant delivery device 10 can be at least partially removed and the second needle 13 can be repositioned.

In some examples, the first needle 12 may be made from metal, for example stainless steel or aluminium. In such examples, as described further with reference to FIG. 26, the first needle 12 may comprise a non-conductive window, for example formed by a plastic insert or the like, to allow wireless communication between an external device and the implant housing 2 while the implant housing 2 is positioned within the first needle 12. This allows the position of the electrode lead to be tested, and also allows the electronics provided in the implant housing 2 to be tested before the implant is deployed.

As shown in FIG. 23C, next the operator retracts the second needle 13 by pushing on the button 35 and actuation tab 16 to move them along the guide member 32 towards the handle 11. As the actuation tab 16 moves along the guide member 32 the rack and pinion mechanism retracts the second needle 13 and releases the electrode lead 3.

In one example, the second needle 13 is only partially retracted in order to expose only an end of the electrode lead 3, where the electrodes (4, see FIG. 1) are located. This partial retraction permits testing of the position of the electrode lead 3 relative to the nerve 9. If such testing shows that the electrode lead is not in the correct location relative to the nerve 9 then the second needle 13 can be pushed back out again to re-sheath the electrode lead 3, and the implant delivery device 10 can be at least partially removed and the second needle 13 can be repositioned.

Once the position of the electrode lead 3 is correct, the second needle 13 is fully retracted to release the electrode lead 3.

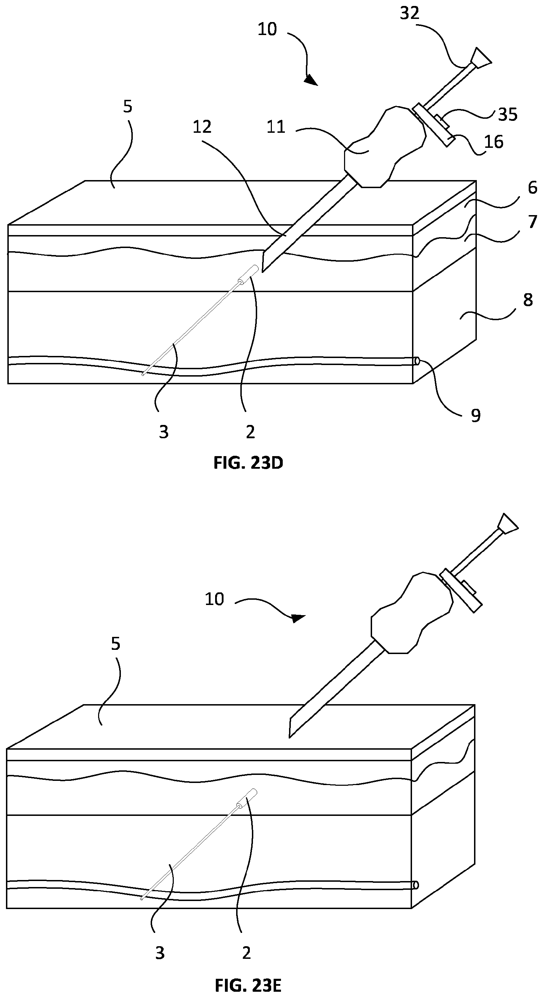

Next, as shown in FIG. 23D, the implant housing 2 is released and deployed from the first needle 12. In some examples, as described with reference to FIGS. 22A and 22B, the release mechanism 39 is operated to release the implant housing 2.

In some examples, friction between the electrode lead 3 and the tissue, in particular the muscle 8, provides a holding force sufficient to pull the implant housing 2 out of the first needle 12 when the implant delivery device 10 is withdrawn from the patient.

Accordingly, the medical implant 1 is deployed, as shown in FIG. 23E, and the implant delivery device 10 can be removed from the patient.

FIG. 24 shows an alternative example second needle 13. In the examples described above the second needle 13 has a lumen in which the electrode lead 3 is received so that the electrode lead 3 extends through the lumen of the second needle 13. The lumen may be open through a side of the second needle 13, akin to a sheath. In the example of FIG. 24 the second needle 13 comprises an attachment point at a distal end 52 of the second needle 13. In this example the attachment point comprises a hole 51 extending at least partially into the second needle 13 at the distal end 52. As illustrated, an end of the electrode lead 3 is received in the hole 51 so that the electrode lead 3 is carried along a side of the second needle 13. The distal end 52 of the second needle 13 also comprises a bevel tip 15. An end of the electrode lead 3 is received in the hole 51, extends through bend 53, and then extends along the side of the second needle 13 to the implant housing received in the first needle (12, see FIG. 3). The bevel tip 15 acts to puncture the patient's skin and tissue and carries the end of the electrode lead 3 to the appropriate depth within the tissue as shown in FIG. 2. The second needle 13 is then retracted, as described above with reference to FIGS. 2 to 23E, leaving the electrode lead 3 in position.

In the example illustrated in FIG. 24 the end of the electrode lead 3 is held in the hole 51 as the second needle 13 is pushed into the patient because of the bend in the electrode lead 3. When the second needle 13 is retracted the end of the electrode lead 3 can slide out of the hole 51, remaining in position.

In some other examples the hole 51 extends to an opposite end of the second needle 13 and a pusher or fluid pressure may be applied to urge the end of the electrode lead 3 out of the hole 51.

In the illustrated example the distal end 52 of the second needle 13 further comprises a notch 54 arranged to receive the bend 53 such that the bevel tip 15 is more fully exposed for puncturing the patient's skin and tissue.

In some examples the second needle 13 alternatively has a hole in a side face of the second needle 13, proximate to the distal end 52. The end of the electrode lead 3 can be received in the hole in the side of the second needle 13 and works in the same way as described above. In a further similar example, the second needle 13 may comprise a socket shaped to receive a matching feature on the electrode lead 3, for example a ball. The socket and matching feature on the electrode lead can attach the electrode lead to the second needle 13 at the attachment point.

It will be appreciated that the example second needle 13 of FIG. 24 may be used in place of the example second needles 13 of any previous example.

In some examples, as shown in FIG. 25, the tip 14 of the first needle 12 comprises a sharp protrusion 56 for piercing the patient's skin and tissue. The first needle 12 with sharp protrusion 56 may be used in any of the example implant delivery devices 10 described herein.

As shown in FIG. 26, in some examples the first needle 12 may include a window 55 corresponding to the position of the implant housing 2 within the first needle 12. The first needle 12 is preferably metal, for example steel or aluminium. The window 55 may be open, for example formed by removing a portion of the first needle 13. Alternatively, the window 55 may comprise an insert, for example a plastic insert. The window 55 allows wireless communication and power between the implant housing 2 and an external device to allow for the medical implant to be tested in situ during deployment into the patient.

In summary, there is provided an implant delivery device for implanting a medical implant in a patient, the medical implant comprising an implant housing and an elongate electrode lead extending from the implant housing. The implant delivery device comprises a handle, a first needle fixed to the handle and extending in a longitudinal direction, and a second needle having a higher gauge than the first needle. The first needle has a lumen adapted to receive the implant housing of the medical implant, and the second needle is adapted to carry the elongate electrode lead of the medical implant. The second needle is retractably mounted to the handle such that the second needle is movable between an extended position and a retracted position. In the extended position the second needle extends beyond the first needle in the longitudinal direction such that during use the first needle and the second needle can simultaneously position the implant housing at a first depth and the electrode lead at a second depth greater than the first depth. In the retracted position the second needle is retracted towards the handle to release the electrode lead from the second needle.

There is also provided a method of implanting a medical implant 1 using the implant delivery device 10. The method includes positioning the first and second needles 12, 13 in the patient to position the medical implant 1 relative to the nerve 9, retracting the second needle 13 to release the electrode lead 3, and then deploying the implant housing 2 and removing the implant delivery device 10, optionally simultaneously.

In some examples the method may include releasing a retaining member to release the implant housing 2 from the first needle 12. In examples, the method may comprise unlocking the second needle 13 from the handle 11 before retracting the second needle 13. In examples, the method may include partially retracting the second needle 13, testing the position of the electrode lead 3 relative to the nerve 9, then re-sheathing the electrode lead 3 and repositioning, if required, before retracting the second needle 13 and deploying the medical implant 1.

Throughout the description and claims of this specification, the words "comprise" and "contain" and variations of them mean "including but not limited to", and they are not intended to (and do not) exclude other components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

### Clauses

Examples of the present disclosure are provided below, in a format of "clauses", noting that such clauses are not to be considered as claims, the claims being appended separately later in this application.
1. An implant delivery device for implanting a medical implant in a patient, the medical implant comprising an implant housing and an elongate electrode lead extending from the implant housing, and wherein the implant delivery device comprises:
   a handle,
   a first needle fixed to the handle and extending in a longitudinal direction, the first needle comprising a lumen adapted to receive the implant housing of the medical implant, and
   a second needle having a higher gauge than the first needle, the second needle being adapted to carry the elongate electrode lead of the medical implant, and wherein the second needle is retractably mounted to the handle such that the second needle is movable between:
      an extended position in which the second needle extends beyond the first needle in the longitudinal direction such that during use the first needle and the second needle can simultaneously position the implant housing at a first depth and the electrode lead at a second depth greater than the first depth, and
      a retracted position in which the second needle is retracted towards the handle to release the electrode lead from the second needle.
2. The implant delivery device of clause 1, wherein the second needle comprises a lumen adapted to receive the elongate electrode lead of the medical implant.
3. The implant delivery device of clause 2, wherein the second needle comprises a slot extending along one side and extending into the lumen.
4. The implant delivery device of clause 3, wherein the first needle and the second needle are non-concentrically arranged such that a portion of the electrode lead extends from the lumen of the second needle through the slot to the implant housing during use.
5. The implant delivery device of clause 1, wherein the second needle comprises an attachment point at or near a distal end of the second needle, and wherein the attachment point is adapted to attach to the elongate electrode lead such that the elongate electrode lead is carried along a side of the second needle.
6. The implant delivery device of clause 5, wherein the attachment point comprises a hole adapted to receive an end of the elongate electrode lead.
7. The implant delivery device of any preceding clause, wherein the second needle is at least partly disposed in the lumen of the first needle.
8. The implant delivery device of clause 7, wherein the lumen of the first needle comprises a primary portion adapted to receive the implant housing and a secondary portion adapted to receive the second needle, the primary portion and the secondary portion each comprising a partially circular cross-section.
9. The implant delivery device of any preceding clause, further comprising a retaining member adapted to releasably secure the implant housing in the lumen of the first needle.
10. The implant delivery device of clause 9, wherein the retaining member comprises a retaining tab formed in a wall of the first needle and adapted to engage the implant housing in the lumen of the first needle.
11. The implant delivery device of clause 9, wherein the implant housing comprises an attachment point, for example a hole, and the retaining member extends from a part of the handle to the attachment point of the implant housing, the retaining member being detachable to release the implant housing.
12. The implant delivery device of clause 11, wherein the retaining member comprises a thread that is looped through the attachment point of the implant housing, and wherein the thread can be split, for example cut or untied, to release the implant housing.
13. The implant delivery device of clause 11, wherein the retaining member comprises a shape memory member having a hooked end adapted to engage the attachment point of the implant housing, and wherein an opposite end of the shape memory housing is arranged to be moved to deform the hooked end to release the implant housing.
14. The implant delivery device of clause 11, wherein the retaining member comprises a clamping cap adapted to be push-fit onto and end of the implant housing.
15. The implant delivery device of clause 14, further comprising a spring-loaded release mechanism operable to separate the clamping cap from the implant housing.
16. The implant delivery device of any preceding clause, further comprising an actuation tab arranged adjacent to the handle, the actuation tab being configured for actuation by a user to move the second needle into the retracted position.
17. The implant delivery device of clause 16, further comprising a locking mechanism adapted to lock the second needle in the extended position.
18. The implant delivery device of clause 17, wherein the locking mechanism comprises a locking slot in the handle, and wherein the second needle is adapted to engage the locking slot such that in one rotational position of the second needle relative to the handle the second needle is locked in the axial direction relative to the handle, and in another rotational position of the second needle relative to the housing the second needle is movable relative to the handle in an axial direction.
19. The implant delivery device of any of clauses 16 to 18, wherein the actuation tab is moveable in a first direction to move the second needle in a second direction opposite to the first direction.
20. The implant delivery device of clause 19, further comprising a rack and pinion mechanism operably coupled between the actuation tab and the second needle.
21. The implant delivery device of any of clauses 16 to 20, wherein the handle comprises a guide member and wherein the actuation tab is movable along the guide member.
22. The implant delivery device of clause 21, wherein the implant delivery device further comprises a locking mechanism is configured to lock the actuation tab at a position along the guide member.
23. The implant delivery device of clause 22, wherein the locking mechanism comprises a button arranged to release the locking mechanism for movement of the actuation tab along the guide member.
24. The implant delivery device of any of clauses 16 to 23, further comprising a retaining member adapted to releasably secure the implant housing in the lumen of the first needle, and wherein the retaining member is actuated by the actuation tab to release the implant housing.
25. The implant delivery device of clause 24, wherein movement of the actuation tab through a first distance in a first direction moves the second needle to the retracted position, and further movement of the actuation tab through a second distance in the first direction actuates the retaining member to release the implant housing.
26. The implant delivery device of clause 24 or clause 25, wherein the retaining member comprises a clamping cap adapted to be push-fit onto the implant housing, and further comprising a spring-loaded release mechanism operable to separate the clamping cap from the implant housing when actuated by the actuation tab.
27. The implant delivery device of any preceding clause, further comprising an implant deployment member comprising a pusher extending from adjacent to the handle into the lumen of the first needle, the implant deployment member being slidable relative to the first needle to push the implant housing out of the first needle.
28. The implant delivery device of any preceding clause further comprising a medical implant comprising an implant housing disposed in the lumen of the first needle and an electrode lead extending from the implant housing and carried by the second needle.

## Claims

1. An implant delivery device for implanting a medical implant in a patient, the medical implant comprising an implant housing and an elongate electrode lead extending from the implant housing, and wherein the implant delivery device comprises:
a handle,
a first needle fixed to the handle and extending in a longitudinal direction, the first needle comprising a lumen adapted to receive the implant housing of the medical implant, and
a second needle having a higher gauge than the first needle, the second needle being adapted to carry the elongate electrode lead of the medical implant, and wherein the second needle is retractably mounted to the handle such that the second needle is movable between:
an extended position in which the second needle extends beyond the first needle in the longitudinal direction such that during use the first needle and the second needle can simultaneously position the implant housing at a first depth and the electrode lead at a second depth greater than the first depth, and
a retracted position in which the second needle is retracted towards the handle to release the electrode lead from the second needle, and
wherein the implant delivery device further comprises a locking mechanism adapted to lock the second needle in the extended position.

2. The implant delivery device of claim 1, wherein the second needle comprises a lumen adapted to receive the elongate electrode lead of the medical implant.

3. The implant delivery device of claim 2, wherein the second needle comprises a slot extending along one side and extending into the lumen.

4. The implant delivery device of claim 3, wherein the first needle and the second needle are non-concentrically arranged such that a portion of the electrode lead extends from the lumen of the second needle through the slot to the implant housing during use.

5. The implant delivery device of claim 1, wherein the second needle comprises an attachment point at or near a distal end of the second needle, and wherein the attachment point is adapted to attach to the elongate electrode lead such that the elongate electrode lead is carried along a side of the second needle.

6. The implant delivery device of any preceding claim, further comprising an actuation tab arranged adjacent to the handle, the actuation tab being configured for actuation by a user to move the second needle into the retracted position.

7. The implant delivery device of claim 6, wherein the handle comprises a guide member and wherein the actuation tab is movable along the guide member, and wherein the locking mechanism is configured to lock the actuation tab at a position along the guide member.

8. The implant delivery device of claim 7, wherein the locking mechanism comprises a button arranged to release the locking mechanism for movement of the actuation tab along the guide member.

9. The implant delivery device of any preceding claim, further comprising an implant deployment member comprising a pusher extending from adjacent to the handle into the lumen of the first needle, the implant deployment member being slidable relative to the first needle to push the implant housing out of the first needle.

10. The implant delivery device of any preceding claim, wherein the second needle is at least partly disposed in the lumen of the first needle.

11. The implant delivery device of any preceding claim, further comprising a retaining member adapted to releasably secure the implant housing in the lumen of the first needle.

12. The implant delivery device of claim 11, wherein the retaining member comprises a retaining tab formed in a wall of the first needle and adapted to engage the implant housing in the lumen of the first needle.

13. The implant delivery device of claim 11, wherein the implant housing comprises an attachment point, for example a hole, and the retaining member extends from a part of the handle to the attachment point of the implant housing, the retaining member being detachable to release the implant housing.

14. The implant delivery device of claim 11, when dependent on any one of claims 6 to 8, wherein the retaining member is actuated by the actuation tab to release the implant housing.

15. The implant delivery device of any preceding claim further comprising a medical implant comprising an implant housing disposed in the lumen of the first needle and an electrode lead extending from the implant housing and carried by the second needle.
